# EUROPÄISCHE PATENTSCHRIFT

(11) **EP 3 687 587 B1**
(45) Veröffentlichungstag und Bekanntmachung des Hinweises auf die Patenterteilung: **07.06.2023**
(21) Anmeldenummer: 18778446.7
(22) Anmeldetag: 24.09.2018
(51) Int. Cl.: A61L 15/10, A61L 15/12

(54) **PASTÖSE ZUBEREITUNG ZUR BILDUNG EINES HALBSTARRVERBANDS**
PASTY PREPARATION FOR FORMING A SEMIRIGID DRESSING
PRÉPARATION PÂTEUSE POUR FORMER UN BANDAGE SEMI-RIGIDE

(30) Priorität: 29.09.2017 DE 102017122705
(43) Veröffentlichungstag der Anmeldung: 05.08.2020
(73) Patentinhaber: KOB GmbH, 67752 Wolfstein (DE)
(72) Erfinder: LANGEN, Günter, 67752 Wolfstein (DE)
(74) Vertreter: DREISS Patentanwälte PartG mbB
(86) Internationale Anmeldenummer: PCT/EP2018/075812
(87) Internationale Veröffentlichungsnummer: WO 2019/063486

(56) Entgegenhaltungen:
- EP-A2- 0 040 378
- CH-A- 282 212
- DE-A1- 10 063 827

## Beschreibung

Die Erfindung betrifft eine pastöse zinkfreie Zubereitung zur Bildung eines Halbstarrverbands. Derartige Halbstarrverbände sind im Stand der Technik als Kompressions-, Stütz- oder Entlastungsverbände bekannt.

Als Halbstarrverbände werden in der Regel Zinkleimverbände, z. B. bei traumatischen oder sportbedingten Verletzungen oder Sehnenscheidenentzündung, eingesetzt. Diese sind seit vielen Jahren auf dem Markt. Derartige Zinkleimverbände bestehen in der Regel aus Zinkoxid, Gelatine, Glycerin und Wasser sowie einem Konservierungsmittel. Daneben können auch andere synthetische oder natürliche wasserlösliche quellbare Polymere eingesetzt werden und die Gelatine ersetzen.

Solche Halbstarrverbände werden in der Regel als Dauerverbände angelegt und haben eine Tragezeit, die von mehreren Tagen bis zu mehreren Wochen reicht. Die Verbände härten dabei über eine gewisse Zeit aus und bilden dann eine vergleichsweise stabile Schale. Im Gegensatz zu Starrverbänden, wie Gipsverbänden, bilden sie jedoch keine biegesteife Struktur aus, sondern behalten eine gewisse Deformierbarkeit.

Dabei werden bei Standard-Zinkleimverbänden Zinkoxid als heilungsfördernder Füllstoff, Gelatine und andere wasserlösliche Polymere als Bindemittel mit Wasser und Glycerin vermischt. Um die Haltbarkeit der Masse zu verlängern, werden weiterhin organische Konservierungsstoffe, z.B. Para-Hydroxy-Benzolsäureester, zugesetzt, um einen mikrobiellen Befall der feucht gelagerten Zinkleimverbände zu verhindern und eine ausreichende Lagerzeit bis zur Verwendung zu ermöglichen.

Zwar wird als Argument für die Verwendung von Zinkleimverbänden häufig die Hautfreundlichkeit genannt, es gibt jedoch Patienten, deren Zahl steigend ist, die durch die enthaltenen Konservierungsmittel zu Hautreizungen neigen. Solche Hautreizungen und Sensibilisierungen und allergischen Reaktionen stellen die gewünschte heilende Wirkung der Zinkleimbinden in Frage, sobald der Verband aufgrund von allergischen Hautreaktionen vorzeitig abgenommen werden muss.

Darüber hinaus kommt es trotz Vorsehung eines Konservierungsmittels immer wieder zu Pilz- oder Bakterienbefall der feucht gelagerten Zinkleimbinden, was sich durch unangenehmen Geruch und unschönes Aussehen bemerkbar macht.

Die EP 0 040 378 schlägt daher vor, Zinkleimhalbstarrverbände mit einer Zusammensetzung aus 10 bis 35% Zinkoxid, 2 bis 15% Zelluloseether, 10 bis 35% Wasser und 0% bis 5% Zusatzstoffen bereitzustellen, wobei insbesondere eine Bestrahlung mit energiereicher Gamma-/Betastrahlung zu Konservierungszwecken erfolgen soll.

Weiterhin ist im Stand der Technik aus der DE 100 63 827 A1 eine Zinkleimbinde bekannt, die
- Wasser, -
- Gelatine oder Alginat oder Hydroxyethylzellulose oder Pektin,
- Glycerin oder Sorbitol,
- Calciumchlorid,
- Zinkoxid,
- Parabene und
- 0,5% bis 5% Dexpanthenol
umfasst und hautpflegende Eigenschaften aufweisen soll.

Aufgabe der Erfindung ist es nun, eine Zubereitung für einen Halbstarrverband, sowie einen Halbstarrverband und ein entsprechendes Set bereitzustellen, das die genannten Nachteile nicht aufweist.

Die Erfindung löst diese Aufgabe durch eine pastöse zinkfreie Zubereitung zur Bildung eines Halbstarrverbandes umfassend folgende Rezeptur:
- 30 bis 70 Gew.-% Wasser
- 1 bis 10 Gew.-% Gelbildner
- 10 bis 40 Gew.-% Glycerin oder mehrwertige Alkohole, insbesondere Propylenglykol und/oder Sorbit
- 0 bis 30 Gew.-% Öl
- 0,2 bis 5 Gew.-% wasserlösliche Salze
- 0,2 bis 2 Gew.-% rheologische Füllstoffe und
- 1 bis 5 Gew.-% adsorbierende Füllstoffe.

Die Erfindung stellt damit eine Zubereitung bereit, bei der es sich um eine zinkfreie Zubereitung handelt, die zusammen mit einem textilen Trägermaterial einen Halbstarrverband zu bilden vermag. Ein derartiger Halbstarrverband dient zur Verwendung als Stütz- oder Kompressionsverband am menschlichen oder tierischen Körper insbesondere nach Sportverletzungen aber auch anderen Traumen, der nach dem Trocknen als Halbstarrverband analog zu den bekannten Zinkleimbinden wirkt, zusätzlich aber Adsorptionseigenschaften für Chemikalien, Gerüche, Hautsekrete und Mikroorganismen aufweist und als solcher in der dermatologischen, phlebologischen oder orthopädischen Therapie eingesetzt werden kann.

Insbesondere weist die Zubereitung folgende Zusammensetzung auf:
- 40 bis 60 Gew.-% Wasser
- 3 bis 7 Gew.-% Gelbildner
- 15 bis 35 Gew.-% Glycerin oder mehrwertige Alkohole, insbesondere Propylenglykol und/oder Sorbit
- 0 bis 20 Gew.-% Öl
- 0,6 bis 2,0 Gew.-% wasserlösliche Salze
- 0,3 bis 1,0 Gew.-% rheologische Füllstoffe und
- 1,5 bis 3,0 Gew.-% adsorbierende Füllstoffe.

Dabei umfasst die pastöse Zubereitung, die durch Vermischen, Lösen und/oder Homogenisierung der Komponenten hergestellt wird, neben Wasser einen Gelbildner, wobei es sich hierbei um wasserlösliche quellbare Polymere, wie insbesondere Gelatine, Stärkederivate, Zelluloseether oder-ester oder Mischungen, Alginate, Guar gum, Acacia, Pektine und/oder Agar Agar oder Kombinationen hiervon handeln kann. Die Gelbildner dienen dabei als Bindemittel und Gerüstpolymer, damit die gewünschte Konsistenz eines Halbstarrverbands erreicht wird.

Glycerin oder andere mehrwertige Alkohole haben dabei die Aufgabe die Härte und Klebrigkeit (Adhäsion und Kohäsion) des Geldbildners zu modifizieren

Weiterhin sind wasserlösliche Salze, insbesondere Natriumchlorid, Kalziumchlorid und/oder Magnesiumchlorid umfasst. Hiermit ist der Vorteil verbunden, dass die Strukturfestigkeit bzw. Konsistenz der pastösen Zubereitung verbessert wird.

Darüber hinaus können Füllstoffe zur Rheologieanpassung (rheologische Füllstoffe) vorgesehen werden, wie insbesondere Aluminiumoxid, Magnesiumoxid und/oder Siliziumdioxid, welche die Viskosität der Zubereitung einstellen.

Die erfindungsgemäß verwendeten Füllstoffe mit Adsorptionseigenschaften (adsorbierende Füllstoffe) sind im Wesentlichen wasserunlösliche, feinpulvrige Stoffe, die aufgrund ihrer hohen inneren Oberfläche eine hohe Bindekapazität für Moleküle bzw. Mikroorganismen (Bakterien, Hefen, Pilze) aufweisen. Dabei ist es gewünscht, dass die Stoffe eine sogenannte "BET"-Oberfläche von 500 bis 1500 m²/g aufweisen. Die Bestimmung des BET-Wertes erfolgt gemäß DIN-ISO 9277:2003-05. Bevorzugt werden hierbei als Füllstoffe mit Adsorptionseigenschaften wie Aktivkohle, Cyclodextrine oder Käfigverbindungen.

Unter Käfigverbindungen oder Clathrate oder, Einschlußverbindungen werden solche Verbindungen verstanden, bei denen Atome oder Moleküle die strukturell bedingten Hohlräume eines geeigneten Molekülgitters besetzen. Dabei sind die "Gastmoleküle" nicht durch chem. Bindungen, sondern durch die Hohlraumstruktur des "Wirtsgitters" in ihrer Lage fixiert. Typische Beispiele sind die festen Gashydrate, z. B. 8 EÂ·46 H₂O (E = Ar, Kr, Xe, Cl₂, CH₄ u. a.), in denen E in Hohlräume eingeschlossen wird, die durch über Wasserstoffbrückenbindungen erfolgende Verknüpfung von (H₂O)₂₀-Dodekaedern gebildet werden. Auch Harnstoff, Cyclodextrine, Hydrochinon, Phenol, Toluol und andere organische Verbindungen, auch einige Koordinationspolymere, bilden geeignete Wirtsgitter.

Weiterhin bevorzugt ist der Einsatz von Metallen oder Metallverbindungen mit antimikrobieller Wirkung, wie beispielsweise Silber oder Kupfer, sollte eine Erhöhung der Lagerstabilität gewünscht sein. Diese werden der Rezeptur bevorzugt mit einem Anteil von 0,05-0,5 Gew.-% zugegeben. Die antimikrobiell wirksamen Metalle können insbesondere in elementarer Form oder als Metallverbindung (Salze, Oxide) eingesetzt werden.

Auf die vorliegende Weise kann ein Halbstarrverband sowie eine Zubereitung hierfür bereitgestellt werden, die sowohl zinkfrei ist, als auch frei von organischen Konservierungsstoffen und damit hautfreundlich. Durch die adsorbierenden Stoffe kann die Zubereitung insbesondere geruchsneutral gestaltet werden, indem Adsorptionseigenschaften für Chemikalien, Gerüche, Hautsekrete, Schweiß und Mikroorganismen vorgesehen sind. Durch den Verzicht auf organische Konservierungsstoffe kann gleichzeitig die Gefahr von Hautreizungen reduziert werden.

Dabei wurde überraschend festgestellt, dass die adsorbierenden Füllstoffe auch konservierende Eigenschaften entfalten, so dass eine gute Lagerstabilität, insbesondere über mehrere Monate oder Jahre hinwegbesteht.

Die Lagerstabilität besteht dabei insbesondere im noch nicht ausgehärteten Zustand, wobei diese durch den Zusatz von Metallen und Metallverbindungen mit antimikrobieller Wirkung, wie beispielsweise Silber oder Kupfer, im Bedarfsfall noch verbessert werden kann.

Besonders bevorzugt ist es dabei, wie bereits dargelegt, wenn es sich um eine zinkfreie Zubereitung handelt. Eine derartige zinkfreie Zubereitung stellt auch aus umwelttoxikologischen Aspekten eine wünschenswerte Gestaltung dar, da der Verband dann schwermetallfrei ist. Klassische Zinkleimbinden enthalten mit ca. 5-35 Gew.-% Zinkoxid einen signifikanten Schwermetallanteil.

Bei der pastösen Zubereitung handelt es sich bevorzugt um eine niedrig- oder mittelviskose pastöse Zubereitung in Form eines Leims, einer Paste oder eines Gels, wobei die Viskosität über die rheologischen Füllstoffe beeinflusst werden kann. Dabei liegt die bevorzugte Viskosität bei 30-6000 mPas bei 60°C. Zur Bestimmung wird ein Brookfield Digital Viskosimeter DV-II+ sowie eine Spindel 1 verwendet (Brookfield, Stoughton, USA).

Die adsorbierenden Füllstoffe werden vorzugsweise gepulvert, vorzugsweise feingepulvert, in der pastösen Zubereitung dispergiert.

Je nach gewünschtem Einsatz können Öle, z.B. paraffinisch und/oder olefinische Öle, z.B Rizinusöl zugegeben werden. Die Geschmeidigkeit des Verbandes wird so auch nach dem Aushärten, erhöht, wobei allerdings, die Lagenhaftung reduziert ist. Solche Verbände werden als "feuchter Typ" bezeichnet.

Dabei ist es besonders bevorzugt, wenn zur Herstellung zunächst die pastöse Zubereitung aus den einzelnen Komponenten inklusive des Füllstoffs mit Adsorptionseigenschaften hergestellt wird und im zweiten Schritt ein Trägertextil mit dieser Zubereitung beschichtet bzw. imprägniert wird. Als Beschichtungstechnik kommen geeignete Beschichtungsverfahren, wie Tauchen, Fouladieren, Pflatschen oder Gießen in Frage, wobei vorzugsweise eine homogene Verteilung der Zubereitung auf das Trägertextil aufgebracht wird.

Bei den verwendeten Füllstoffen mit Adsorptionseigenschaften handelt es sich insbesondere um solche wasserunlöslichen feinpulvrigen Stoffe, die folgende Merkmale aufweisen:
- im Wesentlichen wasserunlösliche, mikroporöse Partikel,
- Wasserlöslichkeit bei 20° C < 1 g/1 l
- innere Oberfläche/BET-Oberfläche im Bereich 500 bis 1500 m²/g
- Partikelfeinheit/Körnung 1 µm bis 100 µm (bevorzugt: 75% Teilchen < 100 µm, besonders bevorzugt mindestens 75% Teilchen < 10 µm)

Dabei wird die Abmessung der Teilchen in üblicher Form bestimmt und bezieht sich auf den größtmöglichen Durchmesser.

Geeignete Füllstoffe mit entsprechenden Adsorptionseigenschaften sind z.B. Aktivkohle, Zeolite und/oder feindisperse Kieselsäure.

Dabei ist es besonders bevorzugt, Aktivkohle als Füllstoff mit Absorptionseigenschaften zu verwenden. In diesem Fall nehmen die Zubereitung sowie der damit hergestellte Halbstarrverband eine dunkelgraue bis schwarze Farbe an, die dann auch nach dem Trocknen im fertig angelegten Halbstarrverband am Patienten so erhalten bleibt. Dieser schwarzgraue Farbton wirkt auf dunkler Haut weniger auffällig als die hochweißen klassischen Zinkleimbinden auf Basis Zinkoxid. Zudem ist die Anschmutzungsempfindlichkeit deutlich verringert, was bei mehrtägiger Tragedauer Vorteile bietet. Darüber hinaus wird die Compliance bei Verbandanwendungen deutlich verbessert, insbesondere, wenn es um geografische Regionen geht, in denen Patienten dunklere Hautfarben aufweisen. Aber auch ohne Zusatz von Aktivkohle und Verwendung anderer absorbierender Füllstoffe weist die Zubereitung in der Regel eine hellgelbe bis beige Farbe auf, die ebenfalls eine geringere Auffälligkeit und Verschmutzungsanfälligkeit besitzt.

Die Erfindung betrifft weiter eine Halbstarrverbandsvorbereitung umfassend einen textilen flachbahnförmigen Träger, auf den die Zubereitung aufgebracht ist. Unter einer Halbstarrverbandsvorbereitung soll die Zusammenstellung aus textilem flachbahnförmigem Träger und Zubereitung verstanden werden, die jedoch noch nicht an einem Patienten angelegt ist. Die Halbstarrverbandsvorbereitung ist dabei das Produkt in seiner noch flexiblen und feuchten Gestaltung. Diese Halbstarrverbandsvorbereitung härtet dann nach Anlegen an einen Patienten oder sonstigem Zutritt von Luft durch Verdunstung der Flüssigkeit, insbesondere des Wassers innerhalb von 12-48 h aus und bildet dann im an einen Patienten angelegten Zustand einen Halbstarrverband. Daneben ist auch ein Halbstarrverbandsset erfindungsgemäß, wobei das Zusammenfügen von Textil und flachbahnförmigem Träger und der Zubereitung zur Bildung eines Halbstarrverbandes erst unmittelbar vor der Anwendung erfolgt.

Als textile Träger kommen Gewebe, Gewirke, Gestricke oder Vliese in Frage. Es ist weiterhin besonders bevorzugt, wenn der textile Träger elastisch ist und insbesondere die Elastizität durch elastische Fäden, insbesondere hochgedrehte Baumwollfäden, als Spin- oder Zwirnkreppfäden, texturierte Polyamid- oder Polyestergarne, Gummi- oder Polyurethanelastanfäden hervorgerufen werden.

Erfindungsgemäß ist auch ein Verfahren zur Herstellung eines Halbstarrverbandes oder einer Halbstarrverbandsvorbereitung in der vorangehend beschriebenen Art.

Dabei ist es besonders bevorzugt, wenn das Gewichtsverhältnis des textilen Trägers zur pastösen Zubereitung im Bereich 1:3 bis 1:6 liegt.

Das beschichtete Flachmaterial (Träger) wird zur Lagerung auf einen zylindrischen Wickelkörper in definierter Länge/Breite vorzugsweise auf einen Kunststoffhülsenkern aufgerollt. Der Kunststoffhülsenkern kann bevorzugt Polyethylen, Polystyrol oder Polyether umfassen oder daraus bestehen. Zur Vermeidung von Trocknungsverlusten durch Verdunsten wird die Halbstarrverbandsvorbereitung in einer hermetisch dicht siegelnden Verpackung, z.B. luft- und wasserdampfdichter Folienbeutel, Peelpackung, Tiefziehpackung, verpackt. Geeignet ist zum Beispiel eine dreilagige Verbundfolie, bei der die erste Lage aus einem 12 µm starken Polyester, die zweite Lage aus Aluminium mit einer Stärke von 6,3 µm und die dritte Lage aus einem Polyethylen mit 60 µm besteht. Die feuchte Halbstarrverbandsvorbereitung berührt im verpackten Zustand die dritte Lage (Polyethylen)der Verbundfolie und ist bis zum Öffnen der Verpackung vor der Verwendung geschützt. Analog zu den handelsüblichen Zinkleimbinden kann so eine Lagerzeit von bis zu drei Jahren erreicht werden.

Die Zubereitung bleibt bis zur Anwendung bestimmungsgemäß feucht und trocknet durch Verdunstung erst nach dem Anlegen des Verbandes bis zur gewünschten halbstarren Konfiguration. Das Aushärten dauert ca. 1-4 Tage, vorzugsweise 12-24 Stunden. Eine Restdeformierbarkeit und Restdehnbarkeit ist im Vergleich zu Starrverbänden, wie Gipsverbänden, noch gegeben. (Definition von Halbstarrverbänden / Starrverbänden in RIEDEL/ TRIEBSCH "Verbandstofffibel", 3. Auflage 1983, Seite 115).

Bevorzugt ist, dass das Wasser wenigstens teilweise aus der Zubereitung innerhalb von 12-48h, vorzugsweise 12-24 verdunstet, wodurch der Verband weniger flexibel ist als vor der Verdunstung.

Weiterhin umfasst die Erfindung einen Halbstarrverband im angelegten Zustand umfassend eine Zubereitung wie beschrieben und einen textilen flachmaterialbahnförmigen Träger, auf den die Zubereitung aufgebracht ist, wobei der Halbstarrverband an einen Patienten angelegt, insbesondere angewickelt ist und durch Verdunstung von Wasser innerhalb von 12-48 h zu einem halbfesten Verband aushärtet. Dabei soll unter halbfesten Verband oder Halbstarrverband ein Verband verstanden werden, der die Bewegung eines Gelenkes reduziert, aber nicht vollständig einschränkt.

Die Erfindung betrifft ebenfalls eine pastöse Zubereitung zur Bildung eines Halbstarrverbandes der vorstehenden Art zur Verwendung in der Behandlung von Zuständen und Erkrankungen ausgewählt aus der Gruppe: Venenthrombosen, Schwellungen, die durch Verletzungen oder Störungen der Venenfunktion verursacht werden, chronische venöse Insuffizienz, einschließlich Ulcus cruris venosum und Phlebödem, Thrombophlebitis, Phlebothrombose, Brüchen des Wadenbeins ohne Fehlstellung, Distorsionen, Luxationen, Tendovaginitis, Weiterbehandlung nach Gipsversorgungen, Abbau posttraumatischer Ödeme, zur schnellen Entstauung und zur Beseitigung chronischer Ödeme, Phlebitiden der tiefen und oberflächlichen Venen, postthrombotischen Zuständen, Nachbehandlung von Knochenbrüchen.

Die Erfindung umfasst weiterhin die Verwendung einer pastösen Zubereitung der vorstehenden Art zur Herstellung eines Halbstarrverbandes zur Behandlung von Venenthrombosen, Schwellungen, die durch Verletzungen oder Störungen der Venenfunktion verursacht werden, chronische venöse Insuffizienz, einschließlich Ulcus cruris venosum und Phlebödem, Thrombophlebitis, Phlebothrombose, Brüchen des Wadenbeins ohne Fehlstellung, Distorsionen, Luxationen, Tendovaginitis, Weiterbehandlung nach Gipsversorgungen, Abbau posttraumatischer Ödeme, zur schnellen Entstauung und zur Beseitigung chronischer Ödeme, Phlebitiden der tiefen und oberflächlichen Venen, postthrombotischen Zuständen, Nachbehandlung von Knochenbrüchen.

Die Erfindung soll im Folgenden anhand von Beispielen näher erläutert werden.

### Beispiel 1 Zinkfreie Leimbinde (Halbstarrverbandsvorbereitung) mit Aktivkohle als adsorbierender Füllstoff (trockener Typ)

Die erfindungsgemäße pastöse Zubereitung wird gemäß folgender Rezeptur hergestellt:

| | |
|---|---|
| - Komp. A 490 g vollentsalztes Wasser | (entspricht 56,9%) |
| - Komp. B 37,5 g Carboxy-methylcellulose | (entspricht 4,4%) |
| - Komp. C 10,0 g Gelatine | (entspricht 1,1%) |
| - Komp. D 300 g Glycerin 85% | (entspricht 34,8%) |
| - Komp. E 6,7 g Calciumchlorid-Dihydrat | (entspricht 0,8%) |
| - Komp. F 17,0 g Aktivkohle | (entspricht 2,0%) |
| - Gesamtmenge 861,2 g | (entspricht 100%) |

In einem mit Rührwerk versehenen beheizbaren Behälter (Volumen 2000 ml) wird die angegebene Menge vollentsalztes Wasser (Komp. A) vorgelegt und auf 40 °C aufgeheizt. Unter mittlerer Rührzahl (1000 U/min) wird die Komponente B als weitere Gelbildner(WALOCEL CRT 30 GA, Hersteller Dow, Walsrode) in Pulverform langsam zugegeben und 30 Minuten verrührt, bis eine klare Lösung entstanden ist. Dann wird Komponente C (Gelita Pharmagelatine Gold Extra 180 Bloom, Hersteller Gelita AG, Eberbach) in Pulverform langsam zugegeben und wiederum 30 Minuten verrührt, bis eine klare Lösung entstanden ist. Dann wird nacheinander Komponente D (Glycerin Pharma 85 %, Hersteller Biesterfeld, Friedrichsthal) und E, als wasserlösliches Salz, (Calciumchlorid- Dihydrat, Hersteller MERCK, Darmstadt) zugegeben. Die Mischung ist bis hierher von hellgelber- beiger Farbe. Schließlich wird die Aktivkohle (Carbopal SC 11 PG, Hersteller Donaucarbon , Frankfurt) als Pulver zugegeben und eingerührt, bis eine homogene tief-schwarze Paste entstanden ist. Diese Paste wird unter ständigem Rühren (Drehzahl 500 U/min) auf 30°C abgekühlt und in dieser Form für die Beschichtung des Trägertextils verwendet. Die Paste erscheint optisch homogen. Über 3 Tage hinweg ist keine Sedimentation erkennbar.

Die Masse aus Beispiel 1 ist über mehrere Wochen (4- 8) hinweg lagerstabil, ohne dass ein mikrobieller Befall (Schimmelbelag an der Oberfläche) sichtbar wäre bzw. sich durch Zersetzungsgeruch zu erkennen gibt.

Zur Herstellung des erfindungsgemäßen Halbstarrverbands (Leimbinde) bzw. einer Halbstarrverbandsvorbereitung wird das nachstehend spezifizierte Trägergewebe verwendet, welches auf einer Bandwebmaschine mit Greifer-Schußfadeneinlegevorrichtung hergestellt wird:
Längselastisches Mullbindengewebe nach DIN 61634 mit festen Webkanten, Typ 564, Breite 10 cm:

| | |
|---|---|
| Materialaufbau Gewebe | 71% Viskose, 29% Polyamid, Leinwandbindung |
| Kettfaden 1/ Kettfaden 2 | 17 tex Viskose / 78dtex f 17 x 2 Polyamid texturiert |
| Fadenzahlen Kettfaden 1 / Kettfaden 2 | 56 / 56 pro 10 cm Breite |
| Schussfaden | 17 dtex Viskose |
| Schussfadendichte | 36 Doppelschuss je 10 cm gedehnt (DIN 61632) |
| Flächengewicht gedehnt | 32 g/m² (DIN 61632) |
| Elastizität | In Längsrichtung (Kettrichtung) |
| Dehnbarkeit/Rückzug nach DIN 61632 | 140% /99% |
| Luftdurchlässigkeit DIN 53887 | 5000-7000 l/m² sec |

Die Beschichtung erfolgt mit einem 2-Walzen- Foulard der Firma Mathis, Oberhasli (CH), wobei eine Walze angetrieben ist und mittels Druckluft gegen die andere gepresst werden kann. Die Paste wird in den Walzenspalt (Zwickel) der beiden horizontal angeordneten Gummiwalzen (Durchmesser 100 mm, Breite 300mm, Gummishorehärte 60°) gefüllt. Dann wird der Luftdruck soweit reduziert, dass ein sichtbarer Spalt (0,5 - 1 mm) zwischen den Walzen entsteht. Das elastische Gewebeband wird von oben durch die Paste im Zwickel in den Walzenspalt geführt, bis es durch den Antrieb der Walzen durchgezogen wird. Unten kommt dann das mit der Paste beschichtete Textilband heraus, welches möglichst spannungsarm und kantengerade auf eine Papphülse als Wickelkern gespult wird. Der Anpressdruck bzw. Spalt wird derart eingestellt, dass sich das Gewichtsverhältnis Textil zu Paste im Bereich 1 : 3 bis 1 : 6 bewegt, je nach gewünschtem Pastengehalt. Nach Erreichen der gewünschten Länge wird das beschichtete Band abgeschnitten und das Bindenende an dem zylindrischen Wickelkörper angelegt. Die so hergestellte grau-schwarze Leimbinde zeigt folgende Merkmale:

| Farbe | Grau-Schwarz |
|---|---|
| Bindenbreite | 10,0 cm |
| Bindendurchmesser (auf 30mm Hülse) | 74 mm |
| Bindenlänge ungedehnt (DIN 61632) | 610 cm |
| Bindenlänge gedehnt (DIN 61632) | 1020 cm |
| Dehnbarkeit (DIN 61632) | 67 % |
| Bindengewicht (ohne Hülse) | 188 g |
| Gewichtsanteil Textil | 32 g |
| Gewichtsanteil Zubereitung | 156 g |
| Gewichtsverhältnis Textil/Zubereitung | 1:4,9 |

Analog zu den bekannten Zinkleimbinden wird die Halbstarrverbandsvorbereitung gemäß Beispiel 1 in eine Schlauchbeutelverpackung aus einer luft- und wasserdampfdichten dreilagigen Barrierefolie (z.B. Hersteller Heyne & Penke, Dassel) hermetisch dicht eingeschweißt und darin bis zur Verwendung gelagert. Auch nach mehreren Wochen ist die Binde verwendbar und nicht mikrobiell befallen.

Zur Anwendung wird die Schlauchbeutelverpackung geöffnet und die Binde mit 50 % Überlappung an einem Probandenbein als Unterschenkel- Kompressionsverband faltenfrei angewickelt. Die Lagen haften sicher aufeinander, insbesondere auch das Bindenende am Untergrund. Im Laufe von 12 bis 24 Stunden härtet die Halbstarrverbandsvorbereitung zum Halbstarrverband aus, wobei sich der Verband außen leicht rauh und trocken anfühlt. Die Lagen sind nach dem Trocknen noch fester miteinander verklebt und der grau-schwarze Farbton erscheint etwas aufgehellter. Der Proband fühlt sich mit dem Verband wohl und empfindet keinerlei Gerüche auch beim Tragen über mehrere Tage.

### Beispiel 2: Zinkfreie Leimbinde mit Siliciumdioxid/ Aktivkohle als adsorbierender Füllstoff (feuchter Typ)

Die erfindungsgemäße pastöse Zubereitung wird gemäß folgender Rezeptur hergestellt:

| | |
|---|---|
| - Komp. A 490 g vollentsalztes Wasser | (entspricht 56,7%) |
| - Komp. B 37,5 g Carboxy-methylcellulose | (entspricht 4,3%) |
| - Komp. C 10,0 g Gelatine | (entspricht 1,1%) |
| - Komp. D 150 g Glycerin 85% | (entspricht 17,4%) |
| - Komp. E 150 g Rizinusöl | (entspricht 17,4%) |
| - Komp. F 6,7 g Calciumchlorid-Dihydrat | (entspricht 0,8%) |
| - Komp. G 2,5 g Siliciumdioxid | (entspricht 0,3%) |
| - Komp. H 17,0 g Aktivkohle | (entspricht 2,0%) |
| - Gesamtmenge 863,7 g | (entspricht 100%) |

Die Herstellung erfolgt analog zu dem Vorgehen gemäß Beispiel 1 mit den gleichen Chemikalien in der Reihenfolge der Komponenten A bis H. Als neue Komponente E wird Ricinusöl (Ricinusöl Pharma, Hersteller Kirsch Pharma, Salzgitter) eingesetzt. Das Ricinusöl wirkt später in dem Verband als Feuchtigkeitsspender und Weichmacher, weshalb man diesen Typ von Leimbinden (Halbstarrverbänden) als "feuchten Typ" bezeichnet. Neben der Aktivkohle wird Siliciumdioxid (Aerosil 200, Hersteller Degussa, Frankfurt) als weiterer Füllstoff mit Adsorptionseigenschaften als Komponente G zugemischt. Bis zum Einmischen der Komponente G ist die Masse milchig- grau/weiß. Nach Zugabe der Aktivkohle (Komponente H) ist eine homogene tief-schwarze Paste entstanden, welche unter ständigem Rühren mit einer Drehzahl von 550 U/min auf 30°C (abgekühlt wird und in dieser Form für die Beschichtung des Trägertextils verwendet wird. Die Paste erscheint optisch homogen; über 3 Tage hinweg ist keine Sedimentation erkennbar.

Die Masse aus Beispiel 2 ist ebenfalls über mehrere Wochen (4-8 Wochen) hinweg lagerstabil, ohne dass ein mikrobieller Befall (Schimmelbelag an der Oberfläche) sichtbar wäre bzw. sich durch Zersetzungsgeruch zu erkennen gibt.

Die Beschichtung erfolgt analog zur Beschreibung in Beispiel 1. Diesmal entsteht wiederum eine grau-schwarze Leimbinde als Halbstarrverbandsvorbereitung mit folgenden Merkmalen:

| Farbe | Grau-Schwarz |
|---|---|
| Bindenbreite | 10,0 cm |
| Bindendurchmesser (auf 30mm Hülse) | 73 mm |
| Bindenlänge ungedehnt (DIN 61632) | 615 cm |
| Bindenlänge gedehnt (DIN 61632) | 990 cm |
| Dehnbarkeit (DIN 61632) | 61 % |
| Bindengewicht (ohne Hülse) | 178 g |
| Gewichtsanteil Textil | 31,5 g |
| Gewichtsanteil Zubereitung | 146,5 g |
| Gewichtsverhältnis Textil/Zubereitung | 1:4,6 |

Analog zu Beispiel 1 wird die Binde in eine Schlauchbeutelverpackung aus einer geeigneten luft- und wasserdampfdichten Barrierefolie hermetisch dicht eingeschweißt und darin bis zur Verwendung gelagert. Auch nach mehreren Wochen ist die Binde verwendbar und nicht mikrobiell befallen.

Zur Anwendung wird die Schlauchbeutelverpackung geöffnet und der Verband mit 50 % Überlappung an einem Probandenbein als Unterschenkel- Kompressionsverband faltenfrei angewickelt. Die Lagen haften diesmal - durch den Ricinusanteil bedingt - weniger fest aufeinander, insbesondere auch das Bindenende bzw. Verbandende. Im Laufe von 12 bis 24 Stunden härtet der Verband zum Halbstarrverband aus, wobei sich der Verband diesmal außen geschmeidig, cremig und feucht anfühlt. Die Lagen sind nach dem Trocknen weniger fest miteinander verklebt als in Beispiel 1, was beim feuchten Typ durchaus gewünscht ist. Der grau-schwarze Farbton erscheint wiederum etwas aufgehellter. Der Proband fühlt sich mit dem Verband wohl und empfindet keinerlei Gerüche auch beim Tragen über mehrere Tage.

### Beispiel 3: Vergleichsbeispiel: Zinkhaltige Leimbinde ohne adsorbierender Füllstoff (trockener Typ)

Als Vergleichsbeispiel wird eine zinkhaltige pastöse Zubereitung ohne Zusatz von Füllstoffen mit Adsorptionseigenschaften gemäß folgender Rezeptur hergestellt:

| | |
|---|---|
| - Komp. A 490 g vollentsalztes Wasser | (entspricht 49,3%) |
| - Komp. B 37,5 g Carboxy-methylcellulose | (entspricht 3,8%) |
| - Komp. C 10,0 g Gelatine | (entspricht 1,0%) |
| - Komp. D 300 g Glycerin 85% | (entspricht 30,2%) |
| - Komp. E 6,7 g Calciumchlorid-Dihydrat | (entspricht 0,7%) |
| - Komp. F 150 g Zinkoxid | (entspricht 15,0%) |
| - Gesamtmenge 994,2 g | (entspricht 100%) |

Die Herstellung erfolgt analog zu dem Vorgehen gemäß Beispiel 1 mit den gleichen Chemikalien in der Reihenfolge der Komponenten Abis E. Als Komponente F wird Zinkoxid (Zinkoxid Pharma, Hersteller Brüggemann KG, Heilbronn) zugesetzt. Diese hochweiße Paste zeigt wird unter ständigem Rühren (Drehzahl 500 U/min ) auf 30 °C abgekühlt und in dieser Form für die Beschichtung des Trägertextils verwendet. Die Paste erscheint optisch homogen; nach 1 Tag ist eine leichte Sedimentation in Gestalt eines Bodensatzes aus Zinkoxid erkennbar. Die Masse des Vergleichsbeispiels bildet nach 1 - 2 Wochen an der Oberfläche einen Schimmelbelag, was auf einen mikrobiellen Befall hindeutet. Dieser gibt sich auch durch einen intensiven fauligen Zersetzungsgeruch zu erkennen.

Daher wurde auf die Beschichtung des Textilträgers zur fertigen Leimbinde (Halbstarrverbandsvorbereitung) verzichtet.

## Patentansprüche

1. Pastöse zinkfreie Zubereitung zur Bildung eines Halbstarrverbands umfassend folgende Rezeptur:
- 30-70 Gew.-% Wasser
- 1-10 Gew.-% Gelbildner
- 10-40 Gew.-% Glycerin oder mehrwertige Alkohole, insbesondere Propylenglycol oder Sorbit,
- 0-30 Gew.-% Öl
- 0,2-5 Gew.-% wasserlösliche Salze
- 0,2-2 Gew.-% rheologische Füllstoffe
- 1-5 Gew.-% adsorbierende Füllstoffe.

2. Zubereitung nach Anspruch 1, **dadurch gekennzeichnet**, die Rezeptur 0,05-0,5 Gew.-% Metalle und/oder Metallverbindungen mit antimikrobieller Wirkung beinhaltet.

3. Zubereitung nach einem der Ansprüche 1 oder 2, **dadurch gekennzeichnet, dass** als Gelbildner wasserlösliche Polymere, wie insbesondere Gelatine, Stärkederivate, Cellulosederivate, Alginate, Acacia, Agar-Agar oder Kombinationen hiervon eingesetzt werden.

4. Zubereitung nach einem der vorangehenden Ansprüche, **dadurch gekennzeichnet, dass** als Öle paraffinische und/oder olefinische Öle, z.B. Rizinusöl verwendet werden.

5. Zubereitung nach einem der vorangehenden Ansprüche, **dadurch gekennzeichnet, dass** als rheologische Füllstoffe Aluminiumoxid, Magnesiumoxid und/oder Siliciumdioxid einsetzbar sind.

6. Zubereitung nach einem der vorangehenden Ansprüche, **dadurch gekennzeichnet, dass** als adsorbierende Füllstoffe Aktivkohle, Cyclodextrin und/oder feindisperse Kieselsäure einsetzbar sind.

7. Zubereitung nach einem der vorangehenden Ansprüche 3-6, **dadurch gekennzeichnet, dass** als antimikrobielle Füllstoffe Silber und/oder Kupfer, insbesondere in elementarer Form oder als Verbindung, nämlich als Oxide und/oder Salze eingesetzt werden.

8. Zubereitung nach einem der vorangehenden Ansprüche, **dadurch gekennzeichnet, dass** die Zubereitung niedrig- oder mittelviskos ist und insbesondere eine Viskosität von 30-6000 mPas bei 60°C aufweist.

9. Zubereitung nach einem der vorangehenden Ansprüche, **dadurch gekennzeichnet, dass** die adsorbierenden Füllstoffe pulverförmig in der pastösen Zubereitung dispergiert sind.

10. Zubereitung nach einem der vorangehenden Ansprüche, **dadurch gekennzeichnet, dass** sie eine gelbliche, beige, graue oder schwarze Farbe aufweist

11. Halbstarrverbandsvorbereitung umfassend einen textilen flachbahnförmigen Träger, auf den eine Zubereitung nach einem der vorangehenden Ansprüche aufgebracht ist.

12. Halbstarrverbandset aus einem textilen flachbahnförmigen Träger und einer Zubereitung nach einem der vorangehenden Ansprüche 1 bis 10 und wobei insbesondere das Gewichtsverhältnis von Träger zu Zubereitung 1:3 bis 1:6 beträgt.

13. Halbstarrverbandsvorbereitung oder Halbstarrverbandsset nach Anspruch 11 oder 12, **dadurch gekennzeichnet, dass** der textile Träger mit der Zubereitung beschichtet oder imprägniert oder beschichtbar oder imprägnierbar ist.

14. Halbstarrverbandsvorbereitung oder Halbstarrverbandsset nach einem der vorangehenden Ansprüche 11 -13, **dadurch gekennzeichnet, dass** der textile Träger ein Gewebe, Gewirk, Gestrick oder Vlies ist.

15. Halbstarrverbandsvorbereitung oder Halbstarrverbandsset nach einem der vorangehenden Ansprüche 11 -14, **dadurch gekennzeichnet, dass** der textile Träger elastisch ist, und insbesondere durch elastische Fäden, insbesondere hochgedrehte Baumwollfäden als Spinn- oder Zwirnkreppfäden, texturierte Polyamid- oder Polyestergarne, Gummi- oder Polyurethanelastanfäden elastifiziert ist.

16. Verwendung einer Halbstarrverbandsvorbereitung oder eines Halbstarrverbandssets nach einem der Ansprüche 11 bis 15 als Kompressions-, Stütz-, oder Entlastungsverband.

17. Verfahren zu Herstellung einer Halbstarrverbandsvorbereitung nach einem der vorangehenden Ansprüche 11-16, soweit auf Anspruch 11 rückbezogen, **dadurch gekennzeichnet, dass** eine Zubereitung nach einem der Ansprüche 1-10 ein oder beidseitig auf ein textiles bahnförmiges Trägermaterial aufgebracht wird.

18. Pastöse Zubereitung zur Bildung eines Halbstarrverbandes nach einem der Ansprüche 1 bis 10 zur Verwendung in der Behandlung von Zuständen und Erkrankungen ausgewählt aus der Gruppe: Venenthrombosen, Schwellungen, die durch Verletzungen oder Störungen der Venenfunktion verursacht werden, chronische venöse Insuffizienz, einschließlich Ulcus cruris venosum und Phlebödem, Thrombophlebitis, Phlebothrombose, Brüchen des Wadenbeins ohne Fehlstellung, Distorsionen, Luxationen, Tendovaginitis, Weiterbehandlung nach Gipsversorgungen, Abbau posttraumatischer Ödeme, zur schnellen Entstauung und zur Beseitigung chronischer Ödeme, Phlebitiden der tiefen und oberflächlichen Venen, postthrombotischen Zuständen, Nachbehandlung von Knochenbrüchen.

19. Verwendung einer pastösen Zubereitung nach einem der Ansprüche 1 bis 10 zur Herstellung eines Halbstarrverbandes zur Behandlung Erkrankungen ausgewählt aus der Gruppe: Venenthrombosen, Schwellungen, die durch Verletzungen oder Störungen der Venenfunktion verursacht werden, chronische venöse Insuffizienz, einschließlich Ulcus cruris venosum und Phlebödem, Thrombophlebitis, Phlebothrombose, Brüchen des Wadenbeins ohne Fehlstellung, Distorsionen, Luxationen, Tendovaginitis, Weiterbehandlung nach Gipsversorgungen, Abbau posttraumatischer Ödeme, zur schnellen Entstauung und zur Beseitigung chronischer Ödeme, Phlebitiden der tiefen und oberflächlichen Venen, postthrombotischen Zuständen, Nachbehandlung von Knochenbrüchen.

## Claims

1. A paste-like zinc-free preparation for forming a semirigid dressing comprising the following formulation:
- 30-70% by weight of water
- 1-10% by weight of gelling agent
- 10-40% by weight of glycerol or polyhydric alcohols, especially propylene glycol or sorbitol
- 0-30% by weight of oil
- 0,2-5% by weight of water-soluble salts
- 0,2-2% by weight of rheological fillers
- 1-5% by weight of adsorbent fillers.

2. The preparation as claimed in claim 1, **characterized in that** the formulation contains 0,05-0,5% by weight of metals and/or metal compounds that have an antimicrobial effect.

3. The preparation as claimed in either of claims 1 or 2, **characterized in that** as gelling agents water-soluble polymers such as in particular gelatin, starch derivatives, cellulose derivatives, alginates, acacia, agar agar or combinations thereof are used.

4. The preparation as claimed in any of the preceding claims, **characterized in that** as oils paraffinic and/or olefinic oils, for example castor oil, are used.

5. The preparation as claimed in any of the preceding claims, **characterized in that** as rheological fillers aluminum oxide, magnesium oxide and/or silicon dioxide are useable.

6. The preparation as claimed in any of the preceding claims, **characterized in that** as adsorbent fillers activated carbon, cyclodextrin and/or finely dispersed silica are useable.

7. The preparation as claimed in any of the preceding claims 3-6, **characterized in that** as antimicrobial fillers silver and/or copper, particularly in elemental form or as a compound, specifically as oxides and/or salts, are used.

8. The preparation as claimed in any of the preceding claims, **characterized in that** the preparation has low or medium viscosity and in particular has a viscosity of 30-6000 mPas at 60°C.

9. The preparation as claimed in any of the preceding claims, **characterized in that** the adsorbent fillers are dispersed in powder form in the paste-like preparation.

10. The preparation as claimed in any of the preceding claims, **characterized in that** it is yellowish, beige, grey or black in color.

11. A semirigid dressing preparation comprising a strip-form textile carrier to which a preparation as claimed in any of the preceding claims is applied.

12. A semirigid dressing set consisting of a strip-form textile carrier and a preparation as claimed in any of the preceding claims 1 to 10, and wherein in particular the weight ratio of carrier to preparation is from 1:3 to 1:6.

13. The semirigid dressing preparation or semirigid dressing set as claimed in claim 11 or 12, **characterized in that** the textile carrier is coated or impregnated or coatable or impregnatable with the preparation.

14. The semirigid dressing preparation or semirigid dressing set as claimed in any of the preceding claims 11-13, **characterized in that** the textile carrier is a woven fabric, knitted fabric, crocheted fabric or nonwoven.

15. The semirigid dressing preparation or semirigid dressing set as claimed in any of the preceding claims 11-14, **characterized in that** the textile carrier is elastic and, in particular, elasticized by elastic threads, in particular high-twist cotton threads in the form of spun or twisted crepe threads, textured polyamide or polyester yarns, rubber threads or polyurethane-elastane threads.

16. The use of a semirigid dressing preparation or of a semirigid dressing set as claimed in any of claims 11 to 15 as a compression, support or relief bandage.

17. A method for producing a semirigid dressing preparation as claimed in any of the preceding claims 11-16, insofar as it refers back to claim 11, **characterized in that** a preparation as claimed in any of claims 1-10 is applied to one or both sides of a strip-form textile carrier material.

18. A paste-like preparation for forming a semirigid dressing as claimed in any of claims 1 to 10 for use in the treatment of states and conditions selected from the following group: venous thrombosis, swelling caused by injuries or impairment of venous function, chronic venous insufficiency, including venous leg ulcers and phlebedema, thrombophlebitis, phlebothrombosis, fractures of the fibula without deformity, distortions, luxations, tendovaginitis, further treatment after plaster casts, reduction of post-traumatic edema, for rapid decongestion and elimination of chronic edema, phlebitis of deep and superficial veins, post-thrombotic states, aftercare of bone fractures.

19. The use of a paste-like preparation as claimed in any of claims 1 to 10 for producing a semirigid dressing for the treatment of conditions selected from the following group: venous thrombosis, swelling caused by injuries or impairment of venous function, chronic venous insufficiency, including venous leg ulcers and phlebedema, thrombophlebitis, phlebothrombosis, fractures of the fibula without deformity, distortions, luxations, tendovaginitis, further treatment after plaster casts, reduction of post-traumatic edema, for rapid decongestion and elimination of chronic edema, phlebitis of deep and superficial veins, post-thrombotic states, aftercare of bone fractures.

## Revendications

1. Préparation pâteuse exempte de zinc destinée à former un bandage semi-rigide, comprenant la formulation suivante :
- 30 à 70 % en poids d'eau
- 1 à 10 % en poids de gélifiant
- 10 à 40 % en poids de glycérol ou de polyalcools, en particulier de propylène glycol ou de sorbitol,
- 0 à 30 % en poids d'huile
- 0,2 à 5 % en poids de sels hydrosolubles
- 0,2 à 2 % en poids de charges rhéologiques
- 1 à 5 % en poids de charges absorbantes.

2. Préparation selon la revendication 1, **caractérisée par le fait que** la formulation contient entre 0,05 et 0,5 % en poids de métaux et/ou de composés métalliques à effet antimicrobien.

3. Préparation selon l'une quelconque des revendications 1 ou 2, **caractérisée par le fait que** l'on utilise en tant que gélifiants des polymères hydrosolubles tels que, en particulier, la gélatine, les dérivés d'amidon, les dérivés de cellulose, les alginates, l'acacia, l'agar-agar ou leurs combinaisons.

4. Préparation selon l'une quelconque des revendications précédentes, **caractérisée par le fait que** l'on utilise comme huiles des huiles paraffiniques et/ou oléfiniques, par exemple l'huile de ricin.

5. Préparation selon l'une quelconque des revendications précédentes, **caractérisée par le fait que** l'oxyde d'aluminium, l'oxyde de magnésium et/ou le dioxyde de silicium peuvent être utilisés en tant charges rhéologiques.

6. Préparation selon l'une quelconque des revendications précédentes, **caractérisée par le fait que** le charbon actif, la cyclodextrine et/ou l'acide silicique finement dispersé peuvent être utilisés en tant que charges absorbantes.

7. Préparation selon l'une quelconque des revendications 3 à 6 précédentes, **caractérisée par le fait que** l'argent et/ou le cuivre, en particulier sous forme élémentaire ou en tant que composé, à savoir comme oxydes et/ou sels, sont utilisés en tant que charges antimicrobiennes.

8. Préparation selon l'une quelconque des revendications précédentes, **caractérisée par le fait que** la préparation est de viscosité faible ou moyenne et présente en particulier une viscosité comprise entre 30 et 6000 mPas à 60 °C.

9. Préparation selon l'une quelconque des revendications précédentes, **caractérisée par le fait que** les charges adsorbantes sont dispersées sous forme de poudre dans la préparation pâteuse.

10. Préparation selon l'une quelconque des revendications précédentes, **caractérisée par le fait que** qu'elle présente une couleur jaunâtre, beige, grise ou noire.

11. Préparation de bandage semi-rigide comprenant un support textile en forme de bande plate sur lequel est appliquée une préparation selon l'une quelconque des revendications précédentes.

12. Ensemble de bandage semi-rigide constitué d'un support textile en forme de bande plate et d'une préparation selon l'une quelconque des revendications 1 à 10 précédentes, et dans lequel, en particulier, le rapport pondéral support sur préparation est compris entre 1:3 et 1:6.

13. Préparation de bandage semi-rigide ou ensemble de bandage semi-rigide selon la revendication 11 ou 12, caractérisé(e) par le fait que le support textile est enduit ou imprégné ou peut être enduit ou imprégné de la préparation.

14. Préparation de bandage semi-rigide ou ensemble de bandage semi-rigide selon l'une quelconque des revendications 11 à 13 précédentes, caractérisé(e) par le fait que le support textile est un tissu, un tissu à mailles, un tricot ou un non-tissé.

15. Préparation de bandage semi-rigide ou ensemble de bandage semi-rigide selon l'une quelconque des revendications 11 à 14 précédentes, caractérisé(e) par le fait que le support textile est élastique et en particulier est élastifié par des fils élastiques, notamment des fils de coton retordus comme fils crêpe de filage ou fils crêpe retors, des fils texturés de polyamide ou de polyester, des fils de caoutchouc ou de polyuréthane élasthanne.

16. Utilisation d'une préparation de bandage semi-rigide ou d'un ensemble de bandage semi-rigide selon l'une quelconque des revendications 11 à 15, en tant que bandage compressif, de soutien ou de soulagement.

17. Procédé de production d'une préparation de bandage semi-rigide selon l'une quelconque des revendications précédentes 11 à 16, dans la mesure où elles dépendent de la revendication 11, **caractérisé par le fait qu'**une préparation selon l'une quelconque des revendications 1 à 10 est appliquée sur une face ou les deux faces d'un matériau de support textile en forme de bande.

18. Préparation pâteuse pour former un bandage semi-rigide selon l'une quelconque des revendications 1 à 10, destiné à être utilisé dans le traitement de conditions et de maladies choisies dans le groupe: la thrombose veineuse, les gonflements qui sont causés par des blessures ou des troubles de la fonction veineuse, l'insuffisance veineuse chronique, y compris l'ulcère de jambe veineux et le phlébo-œdème, la thrombophlébite, la phlébothrombose, les fractures du péroné sans déformation, les distorsions, les luxations, la tendovaginite, le traitement ultérieur après plâtre, la réduction d'oedèmes post-traumatiques, pour une décongestion rapide et l'élimination d'oedèmes chroniques, les phlébites des veines profondes et superficielles, les conditions post-thrombotiques, le traitement ultérieur de fractures osseuses.

19. Utilisation d'une préparation pâteuse selon l'une quelconque des revendications 1 à 10, pour la production d'un bandage semi-rigide destiné au traitement de maladies choisies dans le groupe: la thrombose veineuse, les gonflements qui sont causés par des blessures ou des troubles de la fonction veineuse, l'insuffisance veineuse chronique, y compris l'ulcère de jambe veineux et le phlébo-œdème, la thrombophlébite, la phlébothrombose, les fractures du péroné sans déformation, les distorsions, les luxations, la tendovaginite, le traitement ultérieur après plâtre, la réduction d'oedèmes post-traumatiques, pour une décongestion rapide et l'élimination d'oedèmes chroniques, les phlébites des veines profondes et superficielles, les conditions post-thrombotiques, le traitement ultérieur de fractures osseuses.
